**Europäisches Patentamt**

**European Patent Office**

**Office européen des brevets**

(19)

(11) Veröffentlichungsnummer: **0 276 730**

**A2**

(12) **EUROPÄISCHE PATENTANMELDUNG**

(21) Anmeldenummer: **88100715.7**

(22) Anmeldetag: **20.01.88**

(51) Int. Cl.⁴: **C12N 15/00**

(30) Priorität: **24.01.87 DE 3702116**

(43) Veröffentlichungstag der Anmeldung:
**03.08.88 Patentblatt 88/31**

(84) Benannte Vertragsstaaten:
**AT BE CH DE ES FR GB GR IT LI LU NL SE**

(71) Anmelder: **BEHRINGWERKE Aktiengesellschaft**
**Postfach 1140**
**D-3550 Marburg 1(DE)**

(72) Erfinder: **Bernard, Hans-Ulrich, Dr.**
**Schweizer Talstrasse 16**
**D-6900 Heidelberg(DE)**
Erfinder: **Oltersdorf, Tilmann, Dr.**
**St. Anna-Gasse 13**
**D-6900 Heidelberg(DE)**
Erfinder: **Seedorf, Klaus, Dr.**
**15A Woodlawn Ave.**
**San Francisco, CA(US)**

(74) Vertreter: **Meyer-Dulheuer, Karl-Hermann, Dr. et al**
**HOECHST Aktiengesellschaft Zentrale**
**Patentabteilung Postfach 80 03 20**
**D-6230 Frankfurt/Main 80(DE)**

(54) **Kassetten-Vektor-System zur Expression offener Leserahmen in eukaryotischen Zellen.**

(57) Ein Kassetten-Vektor-System wird beschrieben, das folgende System enthält:

a) ein Segment aus einem E. coli-Plasmid mit einem in E. coli wirksamen Replikationsursprung und Selektionsmarker,

b) in höheren eukaryotischen Zellen wirksamen, austauschbare Segmente, nämlich

b¹) einen Promotor,
b²) einen Translationsinitiator,
b³) einen Polylinker,
b⁴) ein Spleißsignal und
b⁵) ein Polyadenylierungssignal, wobei zwei dieser austauschbaren Segmente auch als gemeinsam austauschbare Einheiten ausgestaltet sein können,

gekennzeichnet durch die DNA-Sequenzen (kodierender Strang)

ATG GAT AGA TCT (1)
ATG GAT AGA TCT C (2)
ATG GAT AGA TCT CC (3)

wobei das ATG-Triplett am 5′-Ende der Sequenzen (1), (2) und (3) das einzige Translations-Startkodon darstellt, das funktionell hinter dem Promotor angeordnet ist, und wobei sich an die Sequenzen (1), (2) und (3) der Polylinker b³) anschließt.

EP 0 276 730 A2

FIG.1

1a.

## Kassetten-Vektor-System zur Expression offener Leserahmen in eukaryotischen Zellen

Die Erfindung betrifft ein vielseitiges Kassetten-Vektor-System, das die Transkription und Translation offener Leserahmen (ORF) in höheren eukaryotischen Zellen ermöglicht, aber auf Grund eines E. coli-Replikationsursprungs die Selektion und Amplifikation in E. coli erlaubt. Die bisher bekannten Systeme zur Herstellung von Fusionsproteine gestatten nicht im gewünschten Maße Vorhersagen über die erhaltenen Fusionsproteine, insbesondere bei der Expression eines ORF auf einer cDNA oder auf viralen oder genomischen Exons ohne Translationsinitiationssignale, insbesondere ohne ATG-Startkodon.

Die Erfindung sei im folgenden anhand der Figuren kurz erläutert:

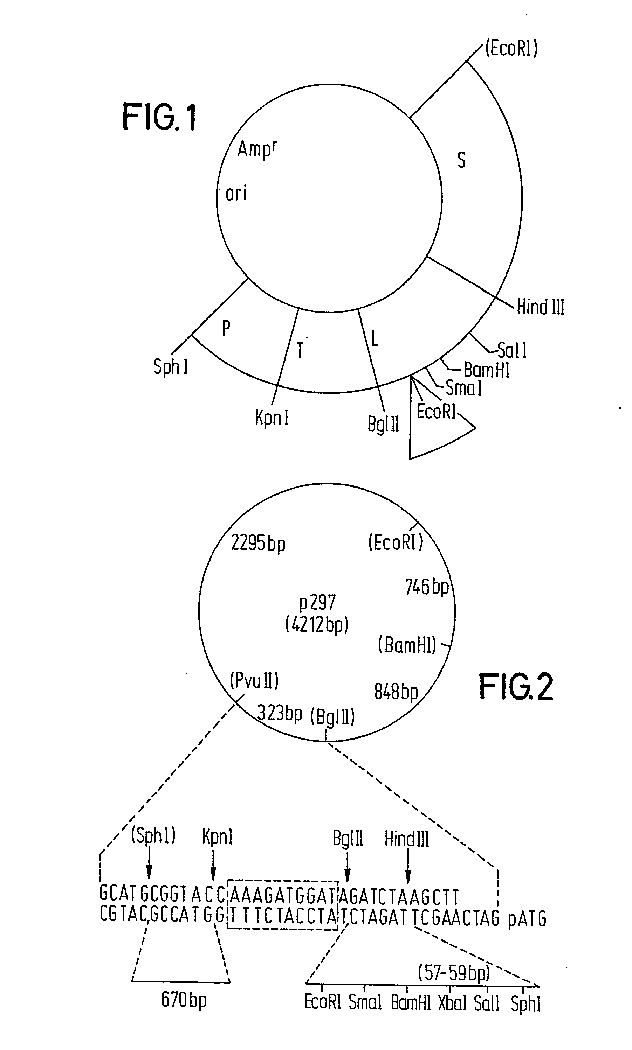

Die Figur 1 zeigt schematisch das erfindungsgemäße Kassetten-Vektor-System, wobei P für Promotor, T für Translations-Initiation, L für eine Polylinkerregion und S für das Spleiß-Polyadenylierungs-Signal steht und wobei der einfache Kresibogen das PvuII-EcoRI-Fragment von pBR322 mit dem Ampicillin-Resistenzgen (Amp$^r$) und dem Replikationsursprung (ori) symbolisiert. Das angedeutete Segment im Bereich der Polylinker-Region soll die einzubauende DNA mit dem zu exprimierenden ORF darstellen.

Die Figur 2 stellt im oberen Bereich das Plasmid p297 dar, in dem die 323 bp-Region durch die darunter angeordnete DNA-Sequenz ersetzt wird, wobei das Plasmid pATG erhalten wird. In dieses Plasmid können - wie beispielhaft angedeutet - an den angegebenen Stellen einerseits ein 670bp-DNA-Fragment mit dem Hyman-Cytomegalovirus (HCMV)-Promotor und andererseits ein 57-59bp-Fragment mit einer Polylinkerregion eingebaut werden, wobei die drei Varianten dieser Polylinkerregion so ausgestaltet sind, daß das ATG-Startkodon innerhalb des umrahmten Bereichs immer in den Leserahmen einer DNA-Sequenz gelangt, die in die Polylinkerregion eingebaut wird.

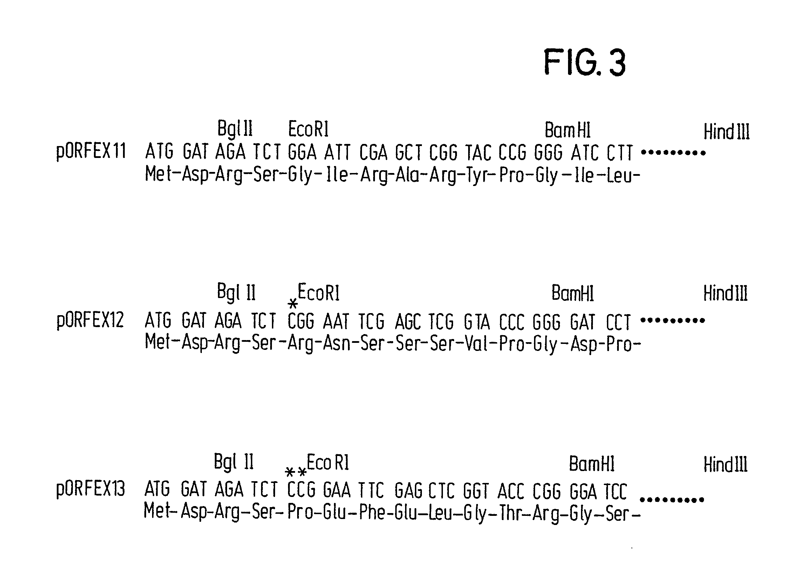

Die Figur 3 zeigt schließlich die DNA-Sequenz der soeben besprochenen Region für die frei Expressionsvektoren pORFEX11-13 und darunter jeweils die entsprechende Aminosäuresequenz, also die N-terminale Region der durch diese Vektoren kodierten Proteine.

Die verschiedenen Aspekte der Erfindung sind in den Patentansprüchen definiert. Im folgenden werden besondere Ausgestaltungen der Erfindung eingehender erläutert.

Zunächst wird die Konsruktion der erfindungsgemäßen Vektoren beschrieben, woraus sich schon die Vielseitigkeit der Abwandlungsmöglichkeiten für den Fachmann ergibt, ohne daß im folgenden hierauf jeweils im einzelnen verwiesen werden muß.

Das 2995bp große pBR322-PvuII-EcoRI-Fragment, das nur beispielhaft für eine geeignete DNA-Sequenz eines E. coli-Plasmids mit Replikationsursprung un Selektionsmarker steht, wird ligiert mit dem 323bp großen SV40-BglII-PvuII-Fragment, das den "early promoter" enthält. Hieran schließt sich das 848bp große SV40-BamHI-BglII-Fragment an, das das Spleiß-Signal der T-Antigenregion enthält. Diese Konstruktion wird komplettiert durch ein 746bp großes SV40-EcoRI-BamHI-Fragment mit der "späten" Polyadenylierungsregion. Das so erhaltene Plasmid p297 (Figur 2) entspricht in seinem Aufbau den bekannten pSV2-Plasmiden (Mulligan und Berg, Science 209 (1980) 1422-1427).

Das Plasmid p297 wird mit BamHI geschnitten, die überstehenden Enden mit Hilfe von Klenow-Polymerase aufgefüllt und die stumpfen Enden unter "blunt end"-Ligierungsbedingungen wieder verbunden. Aus diesem Plasmid mit der eliminierten BamHI-Stelle wird nun das PvuII-BglII-Fragment mit dem SV40-Promotor durch ein synthetisches 34bp-umfassendes Oligonukleotid ersetzt, das in der Figur 2 dargestellt ist. Man erhält so das Plasmid pATG. Die in der Figur eingerahmte Region von 10bp entspricht der SV40-T-Antigen-Startregion.

Im Plasmid pATG läßt sich die Region von der SphI-bis zur KpnI-Schnittstelle durch beliebige eukaryotische Promotoren ersetzen. Am einfachsten geht man hierbei so vor, daß man ein Fragment mit einem solchen Promotor beiderseits stumpfendig macht, beispielsweise durch Auffüllen mit Hilfe von Klenow-Polymerase, worauf das stumpfendige Fragment in die SmaI-Schnittstelle des Vektors pUC18 oder pUC19 eingesetzt und dann als SphI-KpnI-Fragment ausgeschnitten und in das entsprechend geschnittene pATG-Plasmid ligiert wird.

Wählt man z.B. den "immediate early promoter" von HCMV (Boshardt et al., Cell 41 (1985) 521-530; EP-A 0 173 177) als 656 bp HincII-AvaII-Fragment (55bp unterhalb der "cap site") und geht wie beschrieben vor, so erhält man den Expressionsvektor pORFEX10.

Eine andere vorteilhafte Ausgestaltung der Erfindung besteht in der Wahl des Hitze-regulierbaren Drosophila "heat shock-protein 70"-Promotors (hsp; Steller, H., Dissertation 1985, Universität Heidelberg) in Form eines 335bp EcoRI-HindIII-Fragments (85bp unterhalb der mRNA-"cap-site"). Durch den vorstehend beschriebenen Einbau in das Plasmid pATG erhält man das Expressionsplasmid pORFEX20.

In den Plasmiden pORFEX10 und 20 können in die BglII-und HindIII-Schnittstellen DNA-Sequenzen engesetzt werden, die einen ORF im richtigen Leserahmen zum vorhandenen ATG-Startkodon haben. Diese Plasmide stellen also erfindungsgemäße Expressionsvektoren dar. Sie dienen darüber hinaus als Ausgangsvektoren für weitere erfindungsgemäße Vektoren, insbesondere solche mit "verschobenen" Leserahmen ("frame shift").

Anstelle des SV40-T-Antigentranslationsinitiators kann selbstverständlich auch eine entsprechende andere Region eingesetzt werden, und zwar vorteilhaft durch Austausch des KpnI-BglII-Fragments innerhalb des synthetischen Oligonukleotids im Plasmid pATG.

Anstelle der BglII-HindIII-Region im synthetischen Oligonukleotid in pATG kann der modifizierte pUC18-Polylinker eingesetzt werden, dessen 5'-Region in der Figur 3 dargestellt ist. Die in Figur 3 wiedergegebenen Sequenzen beginnen mit dem ATG-Startkodon, das in Figur 2 innerhalb des umrahmten Bereichs liegt. Die BglII-Schnittstellen der Figur 3 sind identisch mit der BglII-Stelle, die in 3'-Richtung benachbart zu dem genannten umrahmten Bereich liegt. Durch Modifizierung der Regionen in Figur 3 zwischen den BglII-und EcoRI-Schnittstellen ergibt sich die stufenweise Verschiebung des Leserahmens, relativ zum Startkodon ATG.

Selbstverständlich steht der modifizierte pUC18-Polylinker nur beispielhaft für eine solche Polylinkerregion, die durch gleichwirkende DNA-Sequenzen ersetzt werden kann. Man kann so - wohl definiert - Proteine mit einem bestimmten N-Terminus konstruieren. Diese Konstruktionen erlauben das Einsetzen von Strukturgenen, die beispielsweise ihr eigenes Startkodon aufweisen. Außerdem lassen sich so bestimmte N-terminale Aminosäuresequenzen kodieren, beispielsweise für Transport-Signale (von Heijne, G., Eur. J. Biochem. 133 (1983) 17-21).

Die erfindungsgemäßen Konstruktionen enthalten kein ATG-Kodon vor dem synthetischen Insert. Hierdurch wird ein sehr effizienter Gebrauch dieses ATG-Tripletts in Eukaryonten gewährleistet. Wird also beispielsweise ein DNA-Fragment in die HindIII-Schnittstelle der Vektoren pORFEX11-13 eingesetzt, so ist eine sichere Aussage möglich, daß die Expression ab dem ATG-Kodon in demjenigen der 3 Vektoren erfolgt, in dem der ORF im Leserahmen mit dem ATG-Kodon liegt.

Eine Voraussetzung für eine Expression ist, daß der Promotor in der richtigen Orientierung eingebaut wurde. Im Falle des HCMV-Promotor-Fragments läßt sich die Orientierung auf Grund der 3 asymmetrisch angeordneten BglI-Stellen und im Falle des hsp-Promotorfragments anhand der XhoI-Schnittstelle überprüfen.

Als zu exprimierendes Strukturgen kann beispielsweise das bakterielle Resistenzgen gegen Hygromycin (Hm$^r$) dienen, sowie ein anderes Gen, das für die bakterielle Chloramphenicol-Acetyltransferase (CAT) kodiert. Besondere Bedeutung haben die erfindungsgemäßen Vektoren jedoch für die Expression von viralen Proteinen, insbesondere von Human-Papillomvirus (HPV)-Proteinen, beispielsweise vom Typ 6, 11, 16 oder 18, die nach Transfektion von Mäuse-oder Human-Zellinien keine oder nur eine ungenaue Transkription ergeben, so daß beispielsweise mit Antikörpern gegen Fusionsproteine aus E. coli keine viralen Proteine gefunden werden können. Wird beispielsweise in die erfindungsgemäßen Vektoren der ORF für das Protein E7 von HPV18 eingebaut, so erhält man nach Transfektion von Maus L-Fibroblasten eine transiente Expression ("transient expression") des E7-Proteins, das in einer Menge von 2 % des Gesamtproteins gefunden wird und als Cytoplasma-Komponente lokalisiert werden kann.

Im Vergleich zu HeLa-Zellen die 10 bis 50 endogene Kopien von HPV18 enthalten (Schwarz et al., Nature 314 (1985) 111-114) wird eine um den Faktor 100 höhere Expression erzielt.

In den folgenden Beispielen wird die Erfindung näher erläutert.


**Beispiel 1**

Aus dem Plasmid pSV2CAT (Gorman et al., Mol. Cell. Biol. 2 (1982) 1044-1051) wird mit HindIII und HpaI das Fragment mit dem CAT-Gen ausgeschnitten, das stromaufwärts 36 weitere Nukleotide enthält und stromabwärts ein Gensegment mit dem SV40-Spleißsignal aufweist (das dem BglII-HpaI-Segment von p297 entspricht).

Der Vektor pORFEX10 wird mit HindIII und HpaI geöffnet und das entsprechende Fragment mit dem CAT-Gen einligiert, wobei das Plasmid pORFEX10-CAT erhalten wird. Dieses hat vor dem CAT-ATG-Startkodon ein weiteres ATG-Kodon, aber in einem anderen Leserahmen. Dieses Plasmid bewirkt deshalb nur eine um den Faktor 20 reduzierte Expression.

Das Plasmid pORFEX10-CAT wird deshalb mit KpnI und HindIII geschnitten und das dabei erhaltene große Fragment mit dem synthetischen Oligonukleotid

AGCTTGTAC

in Gegenwart von dATP ligiert. Man erhält so das Plasmid pORFEX10-CAT-Δ ATG. Dieses Plasmid bewirkt - im Vergleich zu pSV2CAT - eine um den Faktor 5 erhöhte CAT-Expression.


## Tabelle 1

| Plasmid | % umgewandeltes Chloramphenicol |
|---|---|
| - | 0 |
| pSV2CAT | 12,4 |
| pORFEX10-CAT | 3,0 |
| pORFEX10-CAT-ΔATG | 60,9 |


Beispiel 2

Bei Bernard et al., Exp. Cell Res. 158 (1985) 237-243, sind BamHI-HindIII-Fragmente beschrieben, die das bakterielle Hygromycin-Resistenzgen (Hm$^r$) in allen drei Leserahmen enthalten. Diese Fragmente werden als BamHI-HindIII-Fragmente in pORFEX10 eingebaut, das mit HindIII und BamHI gechnitten wurde. Transfektion in L-Zellen ergibt nach 2 Wochen und Selektion gegen Hygromycin nur im Falle des Klons pORFEX10-Hm24 viele gut ausgebildete Kolonien, die dem Ausgangsplasmid pHMR272 (Bernard et al., a.a.O.) entsprechen. Die wenigen kleinen Kolonien im Falle des Klons pORFEX10-Hm19 sind auf ein zweites ATG-Kodon außerhalb des Leserahmens zurückzuführen. Die Ergebnisse zeigt die Tabelle 2.


## Tabelle 2

| Plasmid | Zahl der Kolonien |
|---|---|
| - | 0 |
| pHMR272 | 290 |
| pORFEX10 | 0 |
| pORFEX10-Hm16 | 0 |
| pORFEX10-Hm19 | 11 (klein) |
| pORFEX10-Hm24 | 220 |
| pORFEX10-Hm24* | 0 |

* umgekehrte Orientierung

**Beispiel 3**

In der deutschen Patentanmeldung P 36 25 257.3 sind ähnliche Vektorkonstruktionen vorgeschlagen, in denen eine Polylinker-Region schrittweise um eine Nukleotidposition verschoben ist, so daß das darin eingesetzte, zu exprimierende Gen zwangsläufig in einer dieser drei Anordnungen im richtigen Leserahmen liegt. Setzt man in den Vektor pEX10-mer den ORF für das HPV18-E7-Protein ein, so kodiert der erhaltene Vektor für ein Fusionsprotein, bei dem an der Übergangsstelle zwischen dem MS2-Protein und dem HPV18-E7-Protein die folgende DNA-Sequenz (kodierender Strang) liegt:

$$\text{CTG AAT TCC GGG GGA TCC GTC .}$$
$$\text{EcoRI} \qquad\qquad \text{BamHI}$$

In der EcoRI-Sequenz ist das Triplett AAT im gleichen Leserahmen wie in pORFEX12, nicht aber in pORFEX11 oder 13. Dasselbe gilt für das TCC-Triplett in der BamHI-Schnittstelle.

Da beim Schneiden mit BamHI das gleiche überstehende Ende resultiert wie mit BglII, können die Vektoren pORFEX10 und pORFEX20 nach Schneiden mit BglII mit der BamHI-geschnittenen Sequenz ligiert werden, wobei die letztere ebenfalls im richtigen Leserahmen angeordnet ist.

Transfiziert man L-Zellen mit den so erhaltenen Vektoren, so erhält man eine "transient expression" der kodierten Fusionsproteins, das den größten Teil des HPV18-E7-Proteins umfaßt. Nach Lyse der Zellen und "Western blot"-Analyse mit Antikörpern, die in der genannten Patentanmeldung vorgeschlagen sind, findet man das erwartete Protein in den Zellen, die mit pORFEX10, 12 und 20 (im letzteren Fall nach "heat shock"), nicht aber in den Zellen, die mit pORFEX11 und 13 infiziert wurden oder in solchen Zellen, die Konstruktionen mit der umgekehrten Orientierung des HCMV-Promotors enthielten.

In HeLa-Zellen mit 10 bis 50 endogenen Kopien von HPV18 wurde mit Hilfe der Kaninchen-Antikörper gegen das HPV18-E7-Fusionsprotein eine kleine Bande für ein 12,5 kDa-Protein gefunden, also in der erwarteten Größenordnung.

Eine quantitative Analyse mit Hilfe der Szintillationszählung der E7-Banden ergab, daß die infizierten L-Zellen mit durchschnittlich 2 % E7-Protein des gesamten Zellproteins die 100-fache Menge im Vergleich zu den HeLa-Zellen enthielten, die nur 0,01 % des Gesamtzellproteins an E7-Protein aufwiesen.

Durch "immunestaining" konnte gezeigt werden, daß das E7-Protein im Cytoplasma lokalisiert ist.

**Verfahrensbedingungen:**

Mäuse-LTk⁻-Fibroblasten wurden in nach Dulbecco modifiziertem Eagle-Medium (DMEM), supplementiert mit 10 % fötalem Kälberserum, 10 mM HEPES-Puffer (pH 7,4) und 100 μg/ml Penicillin/Streptomycin kultiviert. Zur "transient" DNA-Transfektion wurden $3 \bullet 10^6$ Zellen in eine Schale von 10 cm ⌀ plattiert, 24 Stunden später 2mal mit DMEM gewaschen und hierzu 10 μg Plamid-DNA in 2 ml DMEM gegeben, das 200 μg/ml DMEM-Dextran (0,5 mDa) und 0,1 mM Chloroquin enthielt. Nach 5-stündiger Inkubation bei 37°C wurden die Zellen 2mal mit DMEM gewaschen und dann 48 Stunden in DMEM mit den vorstehend genannten Supplementen kultiviert. Falls ein Plasmid mit dem hsp-Promotor eingesetzt wurde, folgte ein "heat shock" von 5 Stunden bei 42° am Ende der Inkubationszeit.

Die Analyse der transfizierten Zellen durch CAT-Assat erfolgte mit 5 μg Protein, die mit 62,5 μCi ¹⁴C-Chloramphenicol umgesetzt wurden (Gorman et al., a.a.O.).

Zur Immunocytochemie wurden die Zellen 24 Stunden nach DNA-Anwendung auf autoklavierte Glas-Objektträger platiert. 24 Stunden später wurden die Objektträger in phosphatgepufferter Kochsalzlösung gewaschen. Die Zellen wurden 5 Minuten in eiskaltem Methanol und 2 Minuten in Aceton fixiert. Die Immunocytochemie erfolgte mit Kaninchen-Antiseren gegen MS2-HPV18-E7-Fusionsprotein (Deutsche Patentanmeldung P 36 25 257.3; Seedorf, K., Dissertation, Universität Heidelberg, 1986) und einem handelsüblichen Detektionssystem. Die Objektträger wurden zunächst 1 Stunde mit dem genannten Antikörper in einer Verdünnng von 1 : 50 bis 1 : 200 inkubiert, 3mal in phosphatgepufferter Kochsalzlösung gewaschen, 1 Stunde mit einem Biotinyl-Antikaninchen-Antikörper (Verdünnung 1 : 50) inkubiert, erneut gewaschen und 1 Stunde mit Streptavidin-Biotinyl-Peroxidasekomplex (Verdünnung 1 : 100) inkubiert. Als Substrat für die Farbreaktion diente 0,02 % 3-Amino-9-ethylcarbazol in 50 mM Acetatpuffer (pH 5,2) mit 0,93 % Wasserstoffperoxid. Alle Inkubationen erfolgten bei 37°C.

**Ansprüche**

1. Kassetten-Vektor-System, enthaltend

a) ein Segment aus einem E. coli-Plasmid mit einem in E. coli wirksamen Replikationsursprung und Selektionsmarker,

b) in höheren eukaryotischen Zellen wirksame, austauschbare Segmente, nämlich

b¹) einen Promotor,

b²) einen Translationsinitiator,

b³) einen Polylinker,

b⁴) ein Spleißsignal und

b⁵) ein Polyadenylierungssignal,

wobei zwei dieser austauschbaren Segmente auch als gemeinsam austauschbare Einheiten ausgestaltet sein können,

gekennzeichnet durch die DNA-Sequenzen (kodierender Strang)

ATG GAT AGA TCT (1)

ATG GAT AGA TCT C (2)

ATG GAT AGA TCT CC (3)

wobei das ATG-Triplett am 5′-Ende der Sequenzen (1), (2) und (3) das einzige Translations-Startkodon darstellt, das funktionell hinter dem Promotor angeordnet ist, und wobei sich an die Sequenzen (1), (2) und (3) der Polylinker b³) anschließt.

2. Vektor-System nach Anspruch 1, dadurch gekennzeichnet, daß das Segment aus dem E. coli-Plasmid im wesentlichen das 2295bp pBR322 PvuII-EcoRI-Fragment ist.

3. Vektor-System nach Anspruch 1 oder 2, dadurch gekennzeichnet, daß der Promotor der hsp-Promotor des Drosophila-"heat-shock"-Protein-70 ist.

Patentansprüche für folgenden Vertragsstaat : ES

1. Gentechnisches Verfahren, in dem ein Kassetten-Vektor-System verwendet wird, enthaltend

a) ein Segment aus einem E. coli-Plasmid mit einem in E. coli wirksamen Replikationsursprung und Selektionsmarker,

b) in höheren eukaryotischen Zellen wirksamen, austauschbare Segmente, nämlich

b¹) einen Promotor,

b²) einen Translationsinitiator,

b³) einen Polylinker,

b⁴) ein Spleißsignal und

b⁵) ein Poladenylierungssigna,

wobei zwei dieser austauschbaren Segmente auch als gemeinsam austauschbare Einheiten ausgestaltet sein können,

gekennzeichnet durch die DNA-Sequenzen (kodierender Strang)

ATG GAT AGA TCT (1)

ATG GAT AGA TCT C (2)

ATG GAT AGA TCT CC (3)

wobei das ATG-Triplett am 5′-Ende der Sequenzen (1), (2) und (3) das einzige Translations-Startkodon darstellt, das funktionell hinter dem Promotor angeordnet ist, und wobei sich an die Sequenzen (1), (2) und (3) der Polylinker b³) anschließt.

2. Verfahren nach Anspruch 1, dadurch gekennzeichnet, daß das Segment aus dem E. coli-Plasmid im wesentlichen das 2995bp pBR322 PvuII-EcoRI-Fragment ist.

3. Verfahren nach Asnpruch 1 oder 2, dadurch gekennzeichnet, daß der Promotor der hsp-Promotor des Drosophila-"heat-shock"-Protein-70 ist.

# FIG.1

(EcoRI)

S

Amp$^r$

ori

Hind III

Sal I
BamHI
Smal
EcoRI

Sph I

P

T

L

Kpn I

Bgl II

# FIG.2

2295bp

(EcoRI)

746bp

p297
(4212bp)

(BamHI)

(PvuII)

848bp

323bp (BglII)

(SphI)    KpnI              BglII    HindIII

GCAT GCGGT ACC AAAGATGGAT AGATCTAAGCTT
CGTAC GCCAT GG TTTCTACCTA TCTAGATTCGAACTAG pATG

670bp

(57-59bp)

EcoRI SmaI BamHI XbaI SalI SphI

# FIG. 3

pORFEX11
```
          Bgl II      EcoRI                              BamHI          Hind III
pORFEX11  ATG GAT AGA TCT GGA ATT CGA GCT CGG TAC CCG GGG ATC CTT ··········
          Met-Asp-Arg-Ser-Gly-Ile-Arg-Ala-Arg-Tyr-Pro-Gly-Ile-Leu-
```

```
          Bgl II     *EcoRI                              BamHI          Hind III
pORFEX12  ATG GAT AGA TCT CGG AAT TCG AGC TCG GTA CCC GGG GAT CCT ··········
          Met-Asp-Arg-Ser-Arg-Asn-Ser-Ser-Ser-Val-Pro-Gly-Asp-Pro-
```

```
          Bgl II    **EcoRI                              BamHI          Hind III
pORFEX13  ATG GAT AGA TCT CCG GAA TTC GAG CTC GGT ACC CGG GGA TCC ··········
          Met-Asp-Arg-Ser-Pro-Glu-Phe-Glu-Leu-Gly-Thr-Arg-Gly-Ser-
```